# EUROPEAN PATENT APPLICATION

(11) **EP 4 635 402 A1**
(43) Date of publication of application: **22.10.2025**
(21) Application number: 23903994.4
(22) Date of filing: 13.12.2023
(51) Int. Cl.: A61B 5/00, A61B 5/372, A61B 5/346, A61B 5/11, G16H 50/20

(54) **METHOD FOR DETECTION AND/OR PREDICTING EPILEPTIC SEIZURE ON BASIS OF CONTINUOUS BIOSIGNAL MEASUREMENT, SYSTEM THEREFOR AND COMPUTER SYSTEM-READABLE RECORDING MEDIUM**

(30) Priority: 13.12.2022 KR 20220173974
(71) Applicant: SK Biopharmaceuticals Co., Ltd., Seongnam-si, Gyeonggi-do 13494 (KR)
(72) Inventor: PARK, Chan Ho, Seongnam-si, Gyeonggi-do 13494 (KR); KIM, Yongwook, Seongnam-si, Gyeonggi-do 13494 (KR)
(74) Representative: Vossius & Partner Patentanwälte Rechtsanwälte mbB
(86) International application number: PCT/KR2023/020538
(87) International publication number: WO 2024/128793

(57) **Abstract**

A method for detecting and/or predicting an epileptic seizure based on continuous bio signal measurement according to an aspect of the present disclosure performed by a computer includes: a continuous composite bio signal monitoring step of receiving a composite bio signal including user's electroencephalography (EEG) and head electrocardiogram (ECG) from a sensor unit, preprocessing the received bio signal including the EEG and the head ECG, and converting the preprocessed bio signal into a digital signal; extracting a bio signal feature including a EEG feature and a head ECG feature from a user's bio signal; inputting the bio signal feature including the extracted EEG feature and head ECG feature to a machine learning algorithm to analyze the bio signal feature; and storing or outputting a detection and/or prediction result of the seizure according to the analysis result.

## Description

### [Technical Field]

The present disclosure relates to a method and system for detecting and/or predicting an epileptic seizure based on continuous bio signal measurement and a computer system-readable recording medium, and more particularly, to a method and system for detecting and/or predicting an epileptic seizure based on continuous bio signal measurement using a machine learning algorithm and a computer system-readable recording medium.

### [Background Art]

An epileptic seizure is a symptom in which a part of brain nerve cells generates excessive current for a short period of time, causing convulsions in the whole body or a part of the body and sudden loss of consciousness, and directly causes physical damage such as brain damage and trauma due to a fall, and may cause secondary accidents when the epileptic seizure occurs while moving in external space or driving. In addition, when the seizure state continues for more than several minutes, the seizure is a serious disease that may lead to coma or death in severe cases. Therefore, when the epileptic seizure occurs, if proper response is not made within a short period of time, the epileptic seizure is a serious disease that may lead to death.

When the epileptic seizure occurs, an excessive current in brain nerve cells that causes the seizure may be observed through electroencephalography (EEG). In addition to the EEG, changes in bio signals such as electrocardiogram (ECG), ballistocardiogram (BCG), and photoplethysmogram (PPG), and motion signals such as accelerometer (ACM) or angular velocity due to body convulsions may be used to detect whether the seizure occurs.

The occurrence of the epileptic seizure refers to a case where the bio signals and the motion signals exceed seizure determination criteria for a certain period of time or a certain number of times. In general, for the EEG in a normal state, an alpha band component (8 to 12 Hz) shows a relatively large form compared to other EEG frequency bands, and the heart rate in adults is maintained at a constant 60 to 100 bpm (beats per minute). However, during the epileptic seizure, it has been known that slow activity, spike waves about 3 times per second, sharp waves, etc., appear in the EEG, the heart rate in adults may exhibit other characteristics, such as increasing by approximately 30 bpm above the normal resting level or increasing by more than 50%, and there is an increase in irregular patterns of an R-R interval. During the epileptic seizure, the body tremors and, in severe cases, sudden death due to convulsions or seizures may occur. Since a patient loses consciousness during the seizure, a patient with epilepsy should be monitored by a guardian or medical staff at all times. To this end, motion sensors or video image analysis technologies that detect the motion of the epileptic seizure have been developed in the past, but may only detect seizures with significant motion, such as generalized tonic-clonic seizures, and there is a large error rate for seizures with stiff bodies or little motion, such as partial seizures, making it difficult to respond immediately.

### [Disclosure]

### [Technical Problem]

The present disclosure attempts to provide a system and method for detecting and/or predicting a seizure capable of analyzing a seizure bio signal more accurately by measuring a continuous composite bio signal to extract electroencephalography (EEG) raw signals and time-frequency features as EEG-related parameters, extracting electrocardiogram (ECG) raw signals and time-frequency features as ECG-related parameters, inputting the parameters and bio signal values to a pre-trained artificial intelligence model, and outputting whether there is a seizure bio signal from the artificial intelligence model.

In addition, the present disclosure attempts to provide a system and method for detecting and/or predicting a seizure based on a bio signal capable of analyzing bio signals such as partial seizures more accurately by applying raw signals and time-frequency features of electroencephalography (EEG) and electrocardiogram (ECG) related to EEG variability and heart rate variability (R-R interval) to an artificial intelligence model.

In addition, the present disclosure attempts to provide a computer-readable recording medium, on which a program for implementing a method for detecting and/or predicting a seizure in a computer system is recorded.

### [Technical Solution]

A system for detecting and/or predicting epilepsy seizures based on continuous bio signal measurement according to an embodiment of the present disclosure detects whether seizures occur by using changes in bio signals such as electroencephalography (EEG) and head electrocardiogram (head ECG) that induce the seizures and motion signals such as accelerometer or angular velocity caused by body convulsions.

According to an aspect of the present disclosure, a method for detecting and/or predicting an epileptic seizure based on continuous bio signal measurement performed by a computer includes: a continuous composite bio signal monitoring step of receiving a composite bio signal including user's electroencephalography (EEG) and head electrocardiogram (ECG) from a sensor unit, preprocessing the received bio signal including the EEG and the head ECG, and converting the preprocessed bio signal into a digital signal; extracting a bio signal feature including a EEG feature and a head ECG feature from a user's bio signal; inputting the bio signal feature including the extracted EEG feature and head ECG feature to a machine learning algorithm to analyze the bio signal feature; and storing or outputting a detection and/or prediction result of the seizure according to the analysis result.

According to another aspect of the present disclosure, a system for detecting and/or predicting an epileptic seizure based on continuous bio signal measurement includes: a continuous composite bio signal monitoring system that includes a sensor unit continuously sensing a composite bio signal including an electroencephalography (EEG) and a head electrocardiogram (ECG) of a user; an arithmetic logic unit that includes an EEG feature extraction unit extracting an EEG feature from an EEG signal detected from the bio signal monitoring system, and a head ECG feature extraction unit extracting a head ECG feature from a head ECG signal detected from the bio signal monitoring system, and inputs a bio signal feature of the EEG feature and the head ECG feature extracted from the EEG feature extraction unit and the head ECG feature extraction unit to a machine learning algorithm to analyze the bio signal feature; and a memory unit that stores whether a seizure occurs and a likelihood value of the seizure according to an analysis result.

According to still another aspect of the present disclosure, a method for detecting and/or predicting an epileptic seizure includes preprocessing a continuous composite bio-analog signal collected from a sensor unit that collects an electroencephalography (EEG), a head electrocardiogram (ECG), a motion, etc., converting, by an A/D conversion unit, the continuous composite bio-analog signal into digital signals, inputting a feature of the measured bio signal extracted by an arithmetic logic unit to a pre-trained artificial intelligence model, and storing the occurrence and likelihood of the seizure output from the artificial intelligence model in a memory unit or outputting the occurrence and likelihood of the seizure to an output unit.

According to still yet another aspect of the present disclosure, there are provided a method and system for detecting and/or predicting an epileptic seizure based on composite bio signal measurement, in which a bio signal monitoring system that measures a composite bio signal including electroencephalography (EEG), head electrocardiogram (head ECG), motion, etc., is provided, and by extracting an EEG raw signal and its time-frequency features as EEG-related parameters, extracting a head ECG raw signal and its time-frequency features as a head ECG-related parameters, inputting a bio signal-related parameter value to an artificial intelligence model such as a pre-trained convolutional neural network (CNN), and outputting whether a seizure occurs from an artificial intelligence model, whether the seizure occurs or the likelihood of the seizure can be analyzed more accurately.

### [Advantageous Effects]

According to a method and system for detecting and/or predicting a seizure based on continuous bio signal measurement, and a computer system-readable recording medium according to an embodiment of the present disclosure, by measuring bio signals such as electroencephalography (EEG), head electrocardiogram (ECG), and motion signals together in continuous composite bio signals, detecting time-frequency features as EEG and head electrocardiogram-related parameters, inputting the EEG, the head electrocardiogram, the motion-related parameters, and the bio signal values to other deep learning models based on pre-trained CNN or DNN, outputting whether there is a seizure bio signal from an AI model, detecting when the analyzed seizure event exceeds a predetermined threshold, more accurately compensating for whether a seizure occurs in the detected part with a motion signal, and notifying an alarm to a user when the seizure status does not change even after the compensation, it is possible to better cope with dangerous situations.

### [Description of the Drawings]

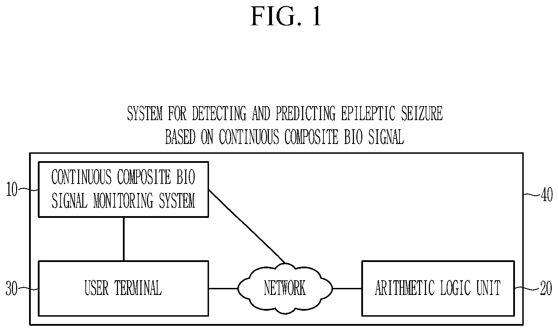

FIG. 1 is a block diagram schematically illustrating a configuration of a system for detecting and/or predicting an epileptic seizure using a composite bio signal according to an embodiment of the present disclosure,
FIG. 2 is a block diagram illustrating a connection relationship between detailed configurations of a continuous composite bio signal monitoring system, an arithmetic logic unit, and a user terminal, which are main components of the system for detecting and/or predicting an epileptic seizure using a composite bio signal according to an embodiment of the present disclosure, and
FIG. 3 is a flowchart illustrating a method for detecting and/or predicting an epileptic seizure using a composite bio signal according to an embodiment of the present disclosure.

### [Mode for Invention]

Hereinafter, a method and system for detecting and/or predicting an epileptic seizure based on continuous bio signal measurement of the present disclosure, and a computer system-readable recording medium are described in detail with reference to the attached drawings.

The method for detecting and/or predicting an epileptic seizure based on continuous bio signal measurement according to an aspect of the present disclosure performed by a computer includes: a continuous composite bio signal monitoring step of receiving a composite bio signal including user's electroencephalography (EEG) and head electrocardiogram (ECG) from a sensor unit, preprocessing the received bio signal including the EEG and the head ECG, and converting the preprocessed bio signal into a digital signal; extracting a bio signal feature including a EEG feature and a head ECG feature from a user's bio signal; inputting the bio signal feature including the extracted EEG feature and head ECG feature to a machine learning algorithm to analyze the bio signal feature; and storing or outputting a detection and/or prediction result of the seizure according to the analysis result.

According to an embodiment of the present disclosure, there are provided a continuous composite bio signal monitoring system that analyzes epileptic seizures more accurately by measuring continuous composite bio signals, extracting EEG, ECG raw signals and time-frequency features as EEG, ECG, and motion-related parameters, inputting the EEG, ECG, and motion-related parameters and bio signal values to a pre-trained artificial intelligence model, and outputting whether an epileptic seizure occurs from the artificial intelligence model, and a seizure detection and/or prediction method based on the same.

According to another aspect of the present disclosure, a system for detecting and/or predicting an epileptic seizure includes: a continuous composite bio signal monitoring system that includes a sensor unit continuously sensing a composite bio signal including an electroencephalography (EEG) and a head electrocardiogram (ECG) of a user; an arithmetic logic unit that includes an EEG feature extraction unit extracting an EEG feature from an EEG signal detected from the bio signal monitoring system, and a head ECG feature extraction unit extracting a head ECG feature from a head ECG signal detected from the bio signal monitoring system, and inputs a bio signal feature of the EEG feature and the head ECG feature extracted from the EEG feature extraction unit and the head ECG feature extraction unit to a machine learning algorithm to analyze the bio signal feature; and a memory unit that stores whether a seizure occurs and a likelihood value of the seizure according to an analysis result.

According to an example of the present disclosure, a seizure refers to a case where a bio signal such as EEG and head ECG, and optionally motion signals exceed seizure determination criteria for a certain period of time or a certain number of times.

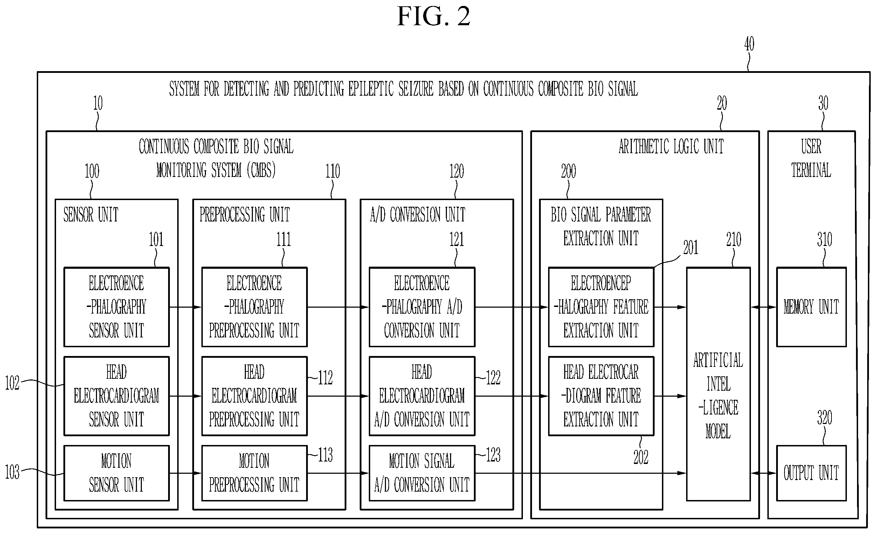

FIG. 2 is a block diagram illustrating a connection relationship between detailed components of a continuous composite bio signal monitoring system, an arithmetic logic unit, and a user terminal, which are main components of the epileptic seizure detection and/or prediction system using a composite bio signal according to an embodiment of the present disclosure, and includes a sensor unit 100, a continuous composite bio signal monitoring system (CMBS) 10 including a preprocessing unit 110 and an A/D conversion unit 120, a bio signal parameter extraction unit 200, an arithmetic logic unit 20 including an artificial intelligence model 210, and a user terminal 30 including a memory unit 310 and an output unit 320.

The continuous composite bio signal monitoring system (CMBS) is a device that measures and provides bio signals and motion signals in real time.

The continuous composite bio signal monitoring system (CMBS) of the present disclosure is composed of an EEG sensor, a head ECG sensor, an optional motion sensor, and a receiver/monitor, and measures bio signals and motion signals in real time to record bio signal data, and stores and outputs the recorded bio signal data to a computer. In particular, the CMBS has a function of notifying with an alarm sound or vibration when there is a significant change in bio signals, such as the occurrence of the epileptic seizure.

The sensor unit 100 may include an EEG sensor unit 101 and a head ECG sensor unit 102, and optionally includes a motion sensor unit 103 and serves to measure the bio signals.

The EEG sensor unit 101 includes an Ag/AgCl medical electrode, and measures the bio signals by mounting the sensor on a patient's forehead, behind the ear, or other exposed skin area with the electrode. Here, the EEG is a signal detected from the potential difference between each measurement site and the reference.

The head ECG sensor unit 102 includes the Ag/AgCl medical electrode and detects the ECG with the electrode. Here, the head ECG is a signal detected from the potential difference behind the right ear and behind the left ear.

The optional motion sensor unit 103 detects three-axis motion using an accelerometer sensor or an angular velocity sensor capable of reflecting motion.

The continuous composite bio-signal monitoring system may further include: an EEG signal preprocessing unit that amplifies an EEG signal detected by an EEG sensor unit and removes noise; and an EEG A/D converter unit that converts an EEG signal received from the EEG signal preprocessing unit into a digital signal.

The continuous composite bio signal monitoring system may further include: a head ECG signal preprocessing unit that amplifies the head ECG signal detected by the head ECG sensor unit and removes noise; and a head ECG A/D converter unit that converts the head ECG signal received from the head ECG preprocessing unit into a digital signal.

The continuous composite bio signal monitoring system may further include a motion signal preprocessing unit that preprocesses the motion bio signal and an A/D conversion unit that converts the preprocessed motion into the digital signal.

The preprocessing unit 110 includes an EEG signal preprocessing unit 111, a head ECG signal preprocessing unit 112, and a motion signal preprocessing unit 113.

The EEG signal preprocessing unit 111 amplifies the bio signal received from the EEG sensor unit 101 and removes noise using an analog filter.

The head ECG preprocessing unit 112 amplifies the bio signal measured by the head ECG sensor unit 102 and removes noise using the analog filter. The ECG is measured differently depending on the direction of application, and representative methods of measuring the ECG include standard limb leads, unipolar limb leads, and chest leads. The standard limb leads are measured using a bipolar lead method by attaching the ECG electrodes to the wearer's right arm, left arm, and left leg. In the standard limb lead, Lead I is recorded by the potential difference between the left arm and right arm, Lead II is recorded by the potential difference between the right arm and left leg, and Lead III is recorded by the potential difference between the left arm and left leg. There is also a method for measuring ECG by connecting electrodes to the back of the ears on the head, and the ECG is recorded by the potential difference of the signal measured from the back of both ears. In this case, there is a previous study that the size of the measured ECG (peak-to-peak amplitude) is approximately 25µV.

The motion signal preprocessing unit 113 may, when necessary, amplify the bio signal measured by the motion sensor unit 103 or remove noise using the analog filter.

The A/D conversion unit 120 converts the bio signal including the EEG and the ECG received from the preprocessing unit 110 into the digital signal and then transmits the bio signal to a bio signal parameter extraction unit 200. The A/D conversion unit 120 also includes an EEG signal A/D conversion unit 121 and a head ECG signal A/D conversion unit 122, and when necessary, a motion signal A/D conversion unit 123. The bio signal may be a simultaneously detected signal.

In an embodiment of the present disclosure, the continuous composite bio signal monitoring system (CMBS) 10 includes a sensor unit 100, a preprocessing unit 110, and an A/D conversion unit 120. That is, the continuous composite bio signal monitoring system (CMBS) 10 is a means for detecting and amplifying a bio signal, removing noise, and converting the bio signal into the digital signal.

The digital signal collected and processed by the continuous composite bio signal monitoring system (CMBS) 10 is transmitted to the bio signal processing unit 20 by directly connecting to the wireless communication module of the user terminal 30 or a wireless communication network through a wireless communication module such as Bluetooth, Wi-Fi, LTE, or 5G built into the device.

In addition, the arithmetic logic unit 20 includes the bio signal parameter extraction unit 200 and an artificial intelligence model.

The bio signal parameter extraction unit 200 includes an EEG feature extraction unit 201 and a head ECG feature extraction unit 202, and receives the bio signal received from the A/D conversion unit 120 to extract parameters of each bio signal and analyze whether a seizure occurs using a raw signal (i.e., the bio signal output from the continuous composite bio signal monitoring system (CMBS)) and the bio signal parameters.

The EEG feature extraction unit 201 receives the bio signal from the EEG A/D conversion unit 121, and the head ECG feature extraction unit 202 receives the bio signal from the head ECG A/D conversion unit 122 to extract the bio signal parameters. The EEG feature extraction unit 201 has a time-frequency feature extraction unit, and the head ECG feature extraction unit 202 has a time-frequency feature extraction unit.

According to one example of the present disclosure, the sensor unit may receive EEG signals measured at two or more head locations and head ECG signals measured at one or more head locations, and is included in an integrated device mounted on the head to sense the bio signal.

In an embodiment of the present disclosure, the bio signal monitoring system that measures the composite bio signal including electroencephalography (EEG), head ECG (head ECG), motion, etc., extracts an EEG raw signal and its time-frequency features as EEG-related parameters, and extracts a head ECG raw signal and its time-frequency features as head ECG-related parameters.

The EEG feature extraction may be calculated by applying short-time Fourier transform (STFT) and wavelet transform methods to EEG of a specific time length (e.g., 10 seconds). There are studies that show that EEG illustrates spike waves or theta waves about 3 times per second as well as gamma waves in a high frequency band during a seizure, and time-frequency features are known as an indicator that may clearly reflect this. From the calculated result, only a power spectral density (PSD) of a specific frequency band (e.g., 0.5 to 50 Hz) is extracted and stored. The head ECG feature extraction analyzes the ECG of the same time length as the EEG feature extraction using the STFT or the wavelet, and extracts and stores only the PSD of the specific frequency band (e.g., 10 to 25 Hz). During the seizure, the sympathetic and parasympathetic nervous systems may be affected by the seizure, causing a breakdown in homeostasis. In particular, when a seizure starts or a seizure wave is transmitted from anterior cingulate cortex, insula, orbitofrontal cortex, and prefrontal cortex, amygdala or hypothalamus may be affected, thereby activating an autonomic nervous system and increasing a heart rate. Since the ECG and the time-frequency characteristics of the ECG are indicators reflecting the heart rate, the ECG and the time-frequency characteristics may also reflect an increase in heart rate.

The EEG features (i.e., STFT or wavelet results) extracted from the EEG feature extraction unit 201, the head ECG features (i.e., STFT or wavelet results) extracted from the head ECG feature extraction unit 202, and the bio signal values output from the continuous composite bio signal monitoring system (CMBS) are applied as inputs to the pre-trained artificial intelligence model 210, and the seizure bio signal prediction result, i.e., whether there is the seizure bio signal (seizure bio signal event-related signal), is calculated with a probability between 0 and 1 and returned as output. Here, the artificial intelligence model 210 may be another model based on CNN and DNN. In addition, the moment when a signal greater than or equal to a threshold has a probability of being a seizure may be identified, and only whether there is the seizure bio signal may be returned as output.

The data used for training the artificial intelligence model may be data prepared at the time of shipment from the factory, or additionally updated data for model correction (calibration).

In other words, the EEG features, the head ECG features, and the bio signal values output from the continuous composite bio signal monitoring system (CMBS) 10 are input to the pre-trained artificial intelligence model 210, and as a result, whether there is the seizure bio signal is output. In this case, the motion signal may be used to assist in determining whether there is the seizure bio signal and calibrate the result.

The user's continuous state (such as walking and sleeping) may be inferred through the motion signal, and even when the result indicating that the seizure bio signal has appeared is derived when there is no change in the continuous state (when the motion signal illustrates the same form as before), this may be considered as a result derived from noise and corrected to not be the seizure.

In an embodiment, the step of inputting the extracted EEG features and head ECG features from a bio signal feature extractor into the machine learning algorithm and analyzing the extracted EEG features and head ECG features is performed by inputting the extracted EEG features and head ECG features to the artificial intelligence model of the pre-trained convolutional neural network to train the bio signals including the EEG and the head ECG, and determining the seizure feature information based on the similarity between the plurality of pieces of feature information, thereby determining the detection and/or prediction results of the seizure.

A composite bio signal arithmetic logic unit includes the EEG feature extraction unit and the head ECG feature extraction unit that extract the time-frequency features from the EEG and head ECG received from the bio signal detection unit using the STFT or wavelet transform method. The composite bio signal arithmetic logic unit processes EEG raw signals and time-frequency features as EEG parameters, head ECG raw signals and time-frequency features as head ECG parameters, and motion raw signals as motion parameters.

The artificial intelligence model according to one example of the present disclosure may be another model based on CNN and a multi-neural network (DNN).

In an embodiment, the memory unit and the output unit of the system for detecting and/or predicting a seizure may be included in the user terminal.

The memory unit 310 and the output unit 320 of the user terminal 30 may receive and store or output the seizure bio signal prediction result from the artificial intelligence model 210. The output unit 320 may be additionally included to output whether the seizure occurs and the probability value of the seizure output from the artificial intelligence model, and may be a display unit or a printer unit.

Here, the bio signal parameter extraction unit 200, the artificial intelligence model 210, the memory unit 310, and the output unit 320 may be formed by a computer or microprocessor, etc. In addition, the EEG sensor unit 101, the EEG preprocessing unit 111, and the EEG A/D conversion unit 121 may be referred to as an EEG extraction unit. The head ECG sensor unit 102, the head ECG signal preprocessing unit 112, and the head ECG signal A/D conversion unit 122 may be referred to as a head ECG extraction unit. The motion sensor unit 103, the motion signal preprocessing unit 113, and the motion signal A/D conversion unit 123 may be referred to as a motion extraction unit.

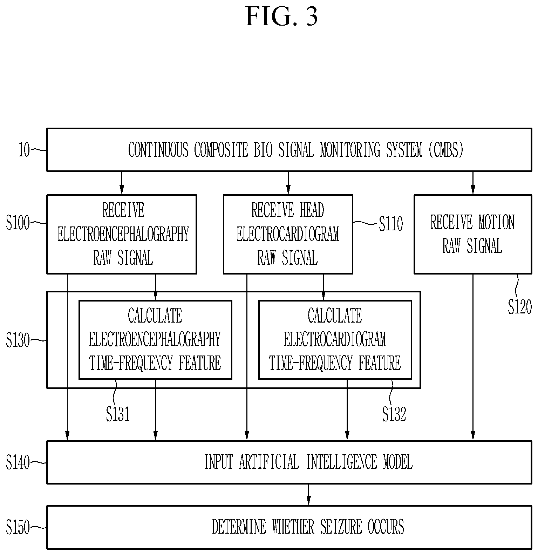

FIG. 3 is a flowchart illustrating a method for detecting and/or predicting an epileptic seizure using a composite bio-signal according to an embodiment of the present disclosure.

The process of analyzing whether the seizure bio signal occurs (whether the seizure bio signal event occurs) in the arithmetic logic unit of FIG. 2 is schematically illustrated.

In the EEG signal receiving step (S100), the bio signal parameter extraction unit 200 receives the EEG from the continuous composite bio signal monitoring system (CMBS) 10.

In the head ECG signal receiving step (S110), the bio signal parameter extraction unit 200 receives the head ECG signal from the continuous composite bio signal monitoring system (CMBS) 10.

In the motion signal receiving step (S120), the bio signal parameter extraction unit 200 receives a motion (e.g., ACM) signal from the continuous composite bio signal monitoring system (CMBS) 10.

In the bio signal feature extraction step (S130), the time-frequency features of the EEG are detected through the EEG time-frequency feature calculation step (S131) from the EEG signal received in the EEG receiving step (S100), and the time-frequency features of the ECG are detected through the head ECG time-frequency feature calculation step (S132) from the head ECG signal received in the ECG receiving step (S110).

When detecting the time-frequency features, the time-frequency features are detected within a moving window of a predetermined time period (e.g., 10 seconds), and the time-frequency features are detected in the order in which the moving window is moved (e.g., 1 second) to detect the next time-frequency features.

In the artificial intelligence model input step (S140), the EEG and head ECG time-frequency features obtained in the EEG time-frequency feature calculation (S131) and the head ECG time-frequency feature calculation (S132) are input to the pre-trained artificial intelligence model 210 (for example, CNN), and the motion signal received in the motion signal receiving step (S120) is input to the artificial intelligence model 210 to correct the result value. In the seizure determination step (S150), the seizure bio signal occurrence value output from the artificial intelligence model 210 is stored in the memory unit 310 and output to the output unit 320.

In addition, the method for predicting a seizure bio signal according to one implementation includes: a parameter detection step in which the arithmetic logic unit detects the time-frequency features from the bio signal received from the bio signal detection unit; a seizure bio signal status analysis step of inputting the time-frequency features and raw signals of the EEG and the head ECG obtained in the parameter detection step to the pre-trained artificial intelligence model, correcting the seizure bio signal occurrence value output from the artificial intelligence model into the motion signal; and storing the corrected seizure bio signal occurrence value in the memory unit or outputting the corrected seizure bio signal occurrence value to the output unit.

### [Industrial Applicability]

In an embodiment of the present disclosure, the step of receiving the seizure detection and/or prediction result output value may use an independent seizure management application.

The present disclosure is not limited to what has been described above and illustrated in the drawings, and it is obvious that those skilled in the art may make many modifications and variations within the scope of the claims described below.

## Claims

1. A method for detecting and/or predicting an epileptic seizure based on continuous bio signal measurement performed by a computer, comprising:
a continuous composite bio signal monitoring step of receiving a composite bio signal including user's electroencephalography (EEG) and head electrocardiogram (ECG) from a sensor unit, preprocessing the received bio signal including the EEG and the head ECG, and converting the preprocessed bio signal into a digital signal;
extracting a bio signal feature including an EEG feature and a head ECG feature from a user's bio signal;
inputting the bio signal feature including the extracted EEG feature and head ECG feature to a machine learning algorithm to analyze the bio signal feature; and
storing or outputting a detection and/or prediction result of the seizure according to the analysis result.

2. The method of claim 1, wherein:
the sensor unit receives an electroencephalography (EEG) signal measured at two or more head locations and a head ECG signal measured at one or more head locations.

3. The method of claim 1, wherein:
the sensor unit is included in an integrated device mounted on a head.

4. The method of claim 1, wherein:
the preprocessing of the received bio signal includes amplifying each measured bio signal and removing noise using an analog filter.

5. The method of claim 1, wherein:
the EEG feature extraction extracts an EEG raw signal and its time-frequency feature.

6. The method of claim 1, wherein:
the head ECG feature extraction extracts a head ECG raw signal and its time-frequency feature as a head ECG parameter.

7. The method of claim 1, wherein:
in the inputting of the bio signal feature including the extracted EEG feature and head ECG feature to the machine learning algorithm to analyze the bio signal , the bio signal feature including the extracted EEG feature and head ECG feature is input to an artificial intelligence model of a pre-trained convolutional neural network to train the bio signal including the EEG and the head ECG, and seizure feature information is determined based on a similarity between a plurality of pieces of feature information to determine detection and/or prediction results of the seizure.

8. The method of claim 1, wherein:
the bio signal further includes a user's motion.

9. A system for detecting and/or predicting an epileptic seizure based on continuous bio signal measurement, comprising:
a continuous composite bio signal monitoring system that includes a sensor unit continuously sensing a composite bio signal including an electroencephalography (EEG) and a head electrocardiogram (ECG) of a user;
an arithmetic logic unit that includes an EEG feature extraction unit extracting an EEG feature from an EEG signal detected from the bio signal monitoring system, and a head ECG feature extraction unit extracting a head ECG feature from a head ECG signal detected from the bio signal monitoring system, and inputs a bio signal feature of the EEG feature and the head ECG feature extracted from the EEG feature extraction unit and the head ECG feature extraction unit to a machine learning algorithm to analyze the bio signal feature; and
a memory unit that stores whether a seizure occurs and a likelihood value of the seizure according to an analysis result.

10. The system of claim 9, wherein:
the sensor unit in the continuous composite bio signal monitoring system is included in an integrated device mounted on a head.

11. The system of claim 9, wherein:
the bio signal further includes a motion signal.

12. The system of claim 9, wherein:
the continuous composite bio signal monitoring system further includes a sensor unit that senses a motion bio signal.

13. The system of claim 9, wherein:
the continuous composite bio signal monitoring system further includes an EEG signal preprocessing unit and a head ECG signal preprocessing unit that preprocess the bio signal including the received EEG and a head ECG.

14. The system of claim 9, wherein:
the continuous composite bio signal monitoring system further includes an A/D conversion unit that converts the bio signal including the preprocessed EEG and the head ECG into a digital signal.

15. The system of claim 9, wherein:
the continuous composite bio signal monitoring system further includes a motion signal preprocessing unit that preprocesses a motion bio signal and an A/D conversion unit that converts the preprocessed motion into a digital signal.

16. The system of claim 9, wherein:
the EEG feature extraction unit extracts the EEG feature including a time-frequency feature from the EEG signal detected from the continuous composite bio signal monitoring system.

17. The system of claim 9, wherein:
the head ECG feature extraction unit extracts a head ECG feature including a time-frequency feature from a head ECG signal detected from the continuous composite bio signal monitoring system.

18. The system of claim 9, wherein:
the continuous processing unit inputs the EEG feature and the head ECG feature extracted from the EEG feature extraction unit and the head ECG feature extraction unit to the machine learning algorithm to train the bio signal and determine seizure feature information based on a similarity between a plurality of pieces of feature information to determine detection and/or prediction results of the seizure.

19. The system of claim 9, further comprising:
an output unit that outputs whether a seizure occurs or a likelihood value of the seizure output from an artificial intelligence model.

20. The system of claim 9, wherein:
the memory unit and the output unit are included in a user terminal.

21. The system of claim 19, wherein:
the artificial intelligence model is a convolutional neural network (CNN) or a deep neural network (DNN).

22. The system of claim 9, wherein:
the continuous composite bio signal monitoring system further includes an EEG signal preprocessing unit that amplifies an EEG signal detected by an EEG sensor unit and removes noise; and an EEG A/D conversion unit that converts an EEG signal received from the EEG signal preprocessing unit into a digital signal.

23. The system of claim 9, wherein:
the continuous composite bio signal monitoring system further includes a head ECG signal preprocessing unit that amplifies the head ECG signal detected by a head ECG sensor unit and removes noise; and a head ECG A/D conversion unit that converts the head ECG signal received from the head ECG preprocessing unit into a digital signal.

24. The system of claim 9, wherein:
the EEG feature extraction unit extracts a power spectral density (PSD) of a specific frequency band among EEGs of a specific time length.

25. The system of claim 9, wherein:
the head ECG extraction unit extracts a power spectral density (PSD) of a specific frequency band among head ECGs of the same time length as that extracted from an EEG feature parameter.

26. The system of claim 9, wherein:
in the receiving of the seizure detection and/or prediction result output value, an independent seizure management application is used.

27. A computer-readable recording medium on which a program for implementing the method for detecting and/or predicting an epileptic seizure based on continuous bio signal measurement as described in any one of claims 1 to 9 is recorded.
